(19) Europäisches Patentamt
European Patent Office
Office européen des brevets

(11) **EP 0 685 232 B1**

(12) **EUROPÄISCHE PATENTSCHRIFT**

(45) Veröffentlichungstag und Bekanntmachung des Hinweises auf die Patenterteilung:
**22.03.2000 Patentblatt 2000/12**

(51) Int. Cl.[7]: **A61K 31/545**, A61K 9/10

(21) Anmeldenummer: **95107930.0**

(22) Anmeldetag: **24.05.1995**

(54) **Cefixim-Zubereitung**

Cefixim formulation

Formulation contenant céfixime

(84) Benannte Vertragsstaaten:
**AT BE CH DE DK ES FR GB GR IE IT LI LU NL PT SE**
Benannte Erstreckungsstaaten:
**LT SI**

(30) Priorität: **31.05.1994 DE 4418957**

(43) Veröffentlichungstag der Anmeldung:
**06.12.1995 Patentblatt 1995/49**

(73) Patentinhaber: **MERCK PATENT GmbH**
**64293 Darmstadt (DE)**

(72) Erfinder:
• **Bolz, Joachim, Dr.**
**D-69121 Heidelberg (DE)**
• **Wagner, Gertraud**
**D-64291 Darmstadt (DE)**
• **Oelrich, Eckhard, Dr.**
**D-64380 Rossdorf (DE)**
• **Radtke, Dirk**
**D-64289 Darmstadt (DE)**

(56) Entgegenhaltungen:
**US-A- 4 079 138          US-A- 5 100 887**

• **BIOPHARMACEUTICS & DRUG DISPOSITION, Bd. 10, Nr. 2, 1989 Seiten 205-211, ROBERT D. FAULKNER ET AL. 'Bioequivalency of oral suspension formulations of cefixime'**

**Beschreibung**

Die Erfindung betrifft pharmazeutische Zubereitungen in Form von nichtwäßrigen Suspensionen, die Cefixim enthalten.

Cefixim ist ein oral anwendbares Cephalosporin-Antibiotikum, das hinsichtlich seiner Struktur, dem Keimspektrum und der Betalaktamasestabilität den parenteral anwendbaren Cephalosporinen der 3. Generation vom Cefotaxim-Typ ähnelt.

Wie alle Vertreter dieser Substanzklasse zeigt es bakterizide Wirkung. Der Wirkungsmechanismus von Cefixim beruht auf einer Hemmung der bakteriellen Zellwandsynthese. Die akute Toxizität von Cefixim ist vernachlässigbar gering.

Der Wirkstoff eignet sich daher zur Behandlung von akuten und chronischen Infektionen unterschiedlichen Schweregrades, die durch Cefiximempfindliche Krankheitserreger verursacht werden und einer oralen Therapie zugänglich sind.

Cefixim wirkt bakterizid und ist beispielsweise gegen folgende Erreger wirksam:

Streptococcus pneumoniae, Streptococcus pyogenes, Streptococcus agalactiae; Hämophilus influenzae, Neisseria gonorrhoeae, Escherichia coli, Proteus mirabilis, Proteus vulguris, Klebsiella pneumoniae, Klebsiella oxytoca, Enterobacter sp., Pasteurella multocida, Providencia sp., Salmonella sp., Shigella sp., Citrobacter amalonaticus, Citrobacter diversus, Serratia marcescens.

Cefixim enthaltende Zubereitungen kamen bislang üblicherweise nur in Form von festen Darreichungsformen wie Tabletten, Kapseln, Granulaten oder Pulvern zur Anwendung. Für die spezielle Wirkungsweise von Cefixim, insbesondere im Hinblick auf einen schnell aufzubauenden Serumspiegel, wären jedoch flüssige Zubereitungen, etwa in Form von Suspensionen, vorteilhaft. Es ist zwar möglich, die handelsüblichen festen Darreichungsformen vor der Anwendung beispielsweise in Wasser aufzuschlämmen und in dieser Form einzunehmen. Diese sind jedoch nur kurzfristig haltbar (max. 2 Wochen). Versuche, Cefixim enthaltende Fertigarzneimittel in Form von Suspensionen herzustellen, schlugen bislang fehl, da mit den üblichen Hilfsstoffen keine auf Dauer stabile Suspensionen zu erhalten waren und der Wirkstoff in wässrigen Lösungen hydrolyseempfindlich ist. In derartigen Suspensionen war der Feststoff außerdem nach kurzer Zeit sedimentiert und derart verklumpt, daß teilweise eine Aufschüttelbarkeit nicht mehr gegeben war. Ferner zeigten diese Suspensionen neben der fehlenden physikalischen Stabilität keine chemische Stabilität, so daß keinerlei Lagerfähigkeit, auch keine kurzzeitige Aufbewahrung, möglich war.

In der U.S.-Patentschrift 4,079,138 wird eine Permanent-Suspension auf nicht-wäßriger Basis als Vehikel für hydrolyseempfindliche Wirkstoffe beschrieben, wobei sämtliche Beispiele auf Amoxicillin und Ampicillin als antibiotische Wirkstoffe gerichtet sind. In diesen Zubereitungen ist ferner, neben anderen Hilfsstoffen, stets ein Verdickungsmittel auf Silikatbasis zugegen.

Ein Zusatz von verdickenden Silikaten führt aber zwangsläufig zu einer starken Erhöhung der Absetzgeschwindigkeit und fördert die Zementierung des Bodensatzes bei abgesetzten Suspensionen, so daß die Aufschüttelbarkeit nicht mehr zufriedenstellend möglich ist.

Zudem wird in diesem Dokument keine Aussage zur Korngrößenverteilung von Wirk- und Füllstoff gemacht, wodurch die Gleichmäßigkeit der Wirkstoffverteilung nicht gewährleistet wird.

Der Erfindung lag somit die Aufgabe zugrunde, ein Verfahren zur Stabilisierung von pharmazeutischen Zubereitungen in Form von Cefixim enthaltenen Suspensionen und eine entsprechende stabile Zubereitung als Fertigarzneimittel zu finden.

Für die Formulierung dieser pharmazeutischen Suspensionen auf nichtwäßriger Basis wird durch geeignete Auswahl der Zusätze eine Sedimentation der suspendierten Partikel verhindert oder zumindest so gering wie möglich gehalten. Hierdurch soll gewährleistet sein, daß das Mittel auch nach längerer Lagerung homogen und entsprechend anwendbar bleibt. Findet doch im Laufe der Zeit eine Sedimentation statt, so soll eine gute Aufschüttelbarkeit des Bodenkörpers gewährleistet sein. Darüber hinaus sollten Suspensionen für die orale Anwendung ein möglichst angenehmes oder zumindest neutrales Geschmacksgefühl vermitteln. Insbesondere muß der Feststoffanteil so eingearbeitet sein, daß dieser bzw. die Feststoffpartikel bei der Einnahme nicht als störend empfunden werden, d.h. es muß für eine hohe Viskosität gesorgt werden. Bei wässrigen Suspensionen können die genannten Eigenschaften üblicherweise durch Zufügen von viskositätserhöhenden Flüssigkeiten wie Glycerin, Propandiol, Sorbitlösung und/oder flüssige Polyethylenglykole erreicht werden. Bei nicht wässrigen Suspensionen kann ein Zusatz von Suspendierungsund Verdickungsmitteln viskositätserhöhend sein. Derartige die Viskosität erhöhende Mittel sind üblicherweise hochmolekulare Cellulosederivate oder Polysaccharidgummen, wie beispielsweise Carboxymethylcellulosen, Methylcellulosen, oder aber Silicate, wie z.B. Carbosil® oder Syloid®. Diese Additive haben aber die zuvor beschriebenen Nachteile, so daß ihr Zusatz keine befriedigende Lösung der Aufgabe liefert, wie in zahlreichen Versuchen, in denen die wesentlichen in der Technologie der pharmazeutischen Suspensionen üblichen Suspendierungs- und Verdickungsmittel getestet wurden, verifiziert werden konnte.

Zur Lösung der Aufgabe, eine stabile, langsam sedimentierende, nichtzementierende Cefixim-Suspension mit gleichzeitig angenehmen Applikationseigenschaften zu entwickeln, ist ein Verzicht auf Verdickungsmittel und eine Gewährleistung einer bestimmten Korngrößenverteilung des Füllmaterials notwendig. Nach Aufschütteln ist eine Gleichmäßigkeit der Wirkstoffverteilung und damit eine korrekte Einzeldosierung - auch bei löffelweiser Entnahme - sichergestellt.

Es wurde nun gefunden, daß sich überraschenderweise stabile Zubereitungen in Form von Cefixim enthaltenden Suspensionen erhalten lassen, wenn man bei diesen Zubereitungen den Wirkstoff in mikronisierter Form sowie feinst gepulvertes Füllmaterial, mit einer Korngrößenverteilung von mindestens 80 Gew.-% kleiner 32 μm und max. 2 Gew.-% größer 50 μm, einsetzt und gleichzeitig auf den Zusatz von verdickenden Hilfsstoffen verzichtet.

Gegenstand der Erfindung sind dementsprechend leicht redispergierbare und chemisch stabile pharmazeutische Zubereitungen in Form von Cefixim enthaltenden nichtwäßrigen Suspensionen, dadurch gekennzeichnet, daß sie

a) frei von verdickenden Hilfsstoffen sind,

b) einen feinstgepulverten Füllstoff mit einer Korngrößenverteilung von mindestens 80 Gew.-% mit einem maximalen Durchmesser von 32 μm und maximal 5 Gew.-% mit einem Durchmesser größer als 50 μm enthalten,

c) 0,1 bis 5 Gew.-% des Wirkstoffes in mikronisierter Form enthalten, der in Triglyceriden mittelkettiger Fettsäuren suspendiert ist,

d) zusätzlich ein Netzmittel in einer Konzentration zwischen 0,01 und 1 Gew.-% enthalten.

In den Zubereitungen ist der Wirkstoff in mikronisierter Form in Konzentrationen zwischen 0,1 und 5 Gew.-%, vorzugsweise zwischen 0,5 und 3, besonders bevorzugt zwischen 1 und 2,5 Gew.-% enthalten.

Ein besonders bevorzugter Wirkstoff ist Cefixim. Darüber hinaus können die erfindungsgemäßen Zubereitungen jeden anderen Wirkstoff, der in dem nichtwäßrigen Medium stabil ist, enthalten. Insbesondere eignen sich jedoch solche, die der Substanzklasse der Cephalosporine angehören, wie z.B. Cephalexin, Cephradin oder Cephotaxim.

Als nichtwäßrige Suspensionsmedien eignen sich ölartige, viskose Flüssigkeiten natürlicher oder synthetischer Herkunft, wie z.B. Erdnuß-oder Sesamöl, Fettsäureester des Polyethylenglykols oder aber Mono-, Di-oder Triglyceride von mittel- bis langkettigen gesättigten oder ungesättigten Fettsäuren. Triglyceridester sind besonders bevorzugt. Insbesondere sind Triglyceride der mittelkettigen Fettsäuren mit 12 bis 20 C-Atomen bevorzugt. Die Konzentrationen der Öle liegen zwischen 40 und 80 Gew.-%, insbesondere aber zwischen 50 und 75, vorzugsweise zwischen 60 und 70 Gew.-%. Es ist auch möglich, Mischungen der natürlichen und/oder synthetischen ölartigen Flüssigkeiten einzusetzen.

Als Füllstoffe eignen sich Zucker oder Zuckerersatzstoffe in feinst gepulverter Form mit einer weitgehend festgelegten Korngrößenverteilung in Bezug auf den Wirkstoff, so daß eine Gleichmäßigkeit der Wirkstoffverteilung sichergestellt ist. Der feinst gepulverte Füllstoff muß so beschaffen sein, daß mindestens 95 Gew.-% der Partikel eine durchschnittliche Korngröße von 50 μm nicht überschreiten und mindestens 80 Gew.-% eine Korngröße kleiner als 32 μm aufweisen. Nur so kann eine ausreichende Viskosität erreicht werden, so daß ein Zusatz von verdickenden Hilfsstoffen entfallen kann und gleichzeitig eine befriedigende physikalische und chemische Stabilität vorliegt.

Eine besonders vorteilhafte Korngrößenverteilung des Füllstoffes liegt dann vor, wenn 50 bis 90 Gew.-% eine Korngröße kleiner 30 μm und 10 bis 50 Gew.-% eine Korngröße zwischen 30 und 50 μm aufweisen, wobei ein Gehalt von maximal 2 Gew.-% mit einer Korngröße zwischen 50 und 100 μm sich nicht nachteilig auf die Stabilität und die Eigenschaften der Suspension auswirkten. Ganz besonders bevorzugt sind Füllmaterialien, bei denen 80 Gew.-% eine Partikelgröße kleiner 32 μm und bis zu maximal 5 Gew.-% eine Teilchengröße größer 50 μm aufweisen, während die übrigen Partikel eine Körnung zwischen 32 und 50 μm besitzen.

Ein bevorzugter Füllstoff ist Saccharose oder der Zuckerersatzstoff Isomalt (Palatinit®). Ferner eignen sich aber auch z.B. Lactose, Glucose, Sorbit, Mannit, Xylit oder Lactit bzw. Mischungen derselben als Füllstoffe, sofern der zuvor angegebenen Korngrößenverteilung Rechnung getragen wird.

Der Anteil der Füllstoffe liegt zwischen 10 und 40 Gew.-%. Bevorzugt ist eine Konzentration zwischen 25 und 35 Gew.-%.

Weitere mögliche Zusatz- und Hilfsstoffe sind beispielsweise Netzmittel, die allerdings nur in Konzentrationen zwischen 0,01 und 1 Gew.-%, vorzugsweise zwischen 0,02 und 0,1 Gew.-% zugegen sein sollten. Als Netzmittel eignen sich die an sich bekannten Tenside, beispielsweise solche, die den Stoffklassen der Alkylbenzolsulfonate, Alkansulfonate, Fettalkoholsulfate, Fettalkoholethersulfate, Fettalkoholethoxylate, Alkylphenolethoxylate oder Alkylphosphonate angehören. Besonders geeignet sind Polysorbate, insbesondere Polysorbat 80.

Andere mögliche Additive sind sogenannte Anticaking-Mittel, wie z.B. Magnesiumstearat, und Aromastoffe zur geschmacklichen Verbesserung der Suspensionen. Diese Zusätze haben aber keine erfindungswesentlichen Effekte und werden in Konzentrationen kleiner 1 Gew.-%, vorzugsweise kleiner 0,25 Gew.-% eingesetzt. Ein Zusatz von Konservierungsmitteln ist nicht erforderlich und übt keinen Einfluß auf Stabilität und Haltbarkeit der erfindungsgemäßen Suspensionen aus.

Die Herstellung der erfindungsgemäßen Suspensionen erfolgt in an sich bekannter Weise durch Mischen der Komponenten und Homogenisieren. Bei Verwendung von Netzmitteln wie Polysorbat werden diese zunächst im hydrophoben Lösungsmittel gelöst oder suspendiert, ehe man die übrigen Komponenten unter ständigem Rühren hinzufügt. Diese können dann in für pharmazeutische Suspensionen übliche Verpackungsmittel wie etwa Flaschen, Trink-Ampullen oder Portionspackungen zur oralen Applikation abgefüllt werden. Der Wirkstoff bleibt über eine lange Lagerzeit (mindestens 5 Jahre) hinweg dispergiert, ohne irreversibel zu sedimentieren, ohne zu verklumpen oder sich an den Gefäßwandungen niederzuschlagen. Er ist nach dieser Lagerzeit durch Aufschütteln sehr leicht suspendierbar.

Der medizinische Anwendungsbereich der erfindungsgemäßen Suspensionen ist völlig analog zu dem der bekannten Darreichungsformen enthaltend Cefixim und/oder andere Cephalosporine. Sie eignen sich zur Behandlung von akuten und chronischen Infektionen unterschiedlichen Schweregrades, die durch Cefixim-empfindliche Krankheitserreger verursacht werden und einer oralen Therapie zugänglich sind; beispielsweise zur Behandlung von:

- Infektionen der oberen und unteren Atemwege

- Infektionen des Hals-Nase-Ohrenbereiches wie z.B. Mittelohrentzündung (Otitis media), Nebenhöhlenentzündungen (Sinusitis), Infektionen der Gaumenmandeln und des Rachenraumes (Tonsillitis, Pharyngitis, Laryngitis)

- Infektionen der Niere und der ableitenden Harnwege

- Infektionen der Gallenwege oder

- akute, gonorrhoischen Urethritis.

Die folgende Dosierung wird empfohlen:

Kinder unter 12 Jahren erhalten täglich 8 mg Cefixim/kg Körpergewicht. Die empfohlene Tagesdosis kann wahlweise auf einmal verabreicht werden oder in 2 Einzeldosen (morgens und abends je 4 mg Cefixim/kg Körpergewicht) aufgeteilt werden. Eine Steigerung der Tagesdosis auf $2 \times 6$ mg Cefixim/kg Körpergewicht ist in Abhängigkeit von der Schwere und der Lokalisation der Infektion möglich.

Erwachsene und Kinder ab 12 Jahren können täglich 400 mg Cefixim erhalten. Die empfohlene Tagesdosis kann wahlweise auf einmal verabreicht werden oder in 2 Einzeldosen (morgens und abends) aufgeteilt werden.

Bei Patienten mit deutlich eingeschränkter Nierenfunktion ist die Dosis zu reduzieren.

Die folgenden Beispiele beschreiben die Erfindung näher, ohne diese jedoch einzuschränken.

Beispiel 1

|  | mg/5 ml | Gew.-% |
|---|---|---|
| Cefixim (mikronisiert) | 100,0 | 1,82 |
| Polysorbat 80® | 2,5 | 0,05 |
| Fruchtaroma (Himbeer) | 4,0 | 0,07 |
| Magnesiumstearat | 8,5 | 0,16 |
| Saccharose (feinst gepulvert) | 1700,0 | 31,03 |
| mittelkettige Triglyceride | 3663,0 | 66,87 |
|  | 5478,0 | 100,00 |

Beispiel 2

|  | mg/5 ml | Gew.-% |
|---|---|---|
| Cefixim (mikronisiert) | 136,6 | 2,50 |
| Polysorbat 80® | 2,5 | 0,05 |
| Fruchtaroma (Erdbeere) | 3,0 | 0,05 |
| Saccharose (feinst gepulvert) | 1650,0 | 30,19 |
| mittelkettige Triglyceride | 3673,0 | 67,21 |
|  | 5465,1 | 100,00 |

Beispiel 3

|  | mg/5 ml | Gew.-% |
|---|---|---|
| Cefixim (mikronisiert) | 100,0 | 1,82 |
| Polysorbat 80® | 2,5 | 0,05 |
| Fruchtaroma (Banane) | 4,0 | 0,07 |
| Magnesiumstearat | 8,5 | 0,16 |
| Isomalt (feinst gepulvert) | 1700,0 | 31,03 |
| mittelkettige Triglyceride | 3663,0 | 66,87 |
|  | 5478,0 | 100,00 |

Beispiel 4

|  | mg/5 ml | Gew.-% |
|---|---|---|
| Cefixim (mikronisiert) | 136,6 | 2,50 |
| Polysorbat 80® | 2,5 | 0,05 |
| Fruchtaroma (Himbeer) | 3,0 | 0,05 |
| Mannit (feinst gepulvert) | 1650,0 | 30,19 |
| mittelkettige Triglyceride | 3673,0 | 67,21 |
|  | 5465,1 | 100,00 |

Die nachfolgenden Darstellungen zeigen die Stabilität der erfindungsgemäßen Suspensionen auf.

Resuspendierbarkeit

Zur Ermittlung der Resuspendierbarkeit wurden Flaschen nach Herstellung und solche nach Lagerung (bis zu 5 Jahren) aufgeschüttelt und 10 Portionen von je 1 ml analysiert (mg Cefixim/5 ml Suspension):

|  | nach Herstellung | nach 60 Monaten |
|---|---|---|
| 1. Entnahme | 97,1 | 95,2 |
| 2. Entnahme | 97,2 | 95,6 |
| 3. Entnahme | 97,3 | 95,4 |
| 4. Entnahme | 97,4 | 95,5 |
| 5. Entnahme | 97,0 | 95,1 |
| 6. Entnahme | 97,0 | 95,3 |
| 7. Entnahme | 97,3 | 95,5 |
| 8. Entnahme | 96,9 | 95,3 |
| 9. Entnahme | 97,7 | 94,9 |
| 10. Entnahme | 97,1 | 95,1 |

Haltbarkeit nach Anbruch

Zur Prüfung der Haltbarkeit nach Anbruch wurden aus Flaschen (à 100 ml) über einen Zeitraum von 14 Tagen täglich 5 bzw. 10 ml Suspension entnommen. Der Cefixim-Gehalt der am 1., 5., 10. und 14. Tag entnommenen Mengen wurde ermittelt:

| 1. Tag | 104,4 mg/5 ml |
|---|---|
| 5. Tag | 105,2 mg/5 ml |
| 10. Tag | 105,1 mg/5 ml |
| 14. Tag | 105,2 mg/5 ml |

Chemische Stabilität

| Lagerung bei 23 °C; 50 % relative Feuchte | | | | |
|---|---|---|---|---|
| **Lagerzeit** | **Aussehen** | **Cefixim** | **Deg. 2** | **Deg. 3** |
| Start | weiße bis schwach gelbliche Suspension | 102,6 mg | 1,1 mg | 0,1 mg |
| 6 Monate | praktisch unverändert | 99,0 mg | 1,4 mg | 0,2 mg |
| 12 Monate | praktisch unverändert | 99,4 mg | 1,9 mg | 0,3 mg |
| 18 Monate | praktisch unverändert | 98,8 mg | 1,8 mg | 0,3 mg |
| 24 Monate | praktisch unverändert | 96,5 mg | 1,8 mg | 0,5 mg |

(fortgesetzt)

| Lagerung bei 23 °C; 50 % relative Feuchte | | | | | |
|---|---|---|---|---|---|
| Lagerzeit | Aussehen | | Cefixim | Deg. 2 | Deg. 3 |
| 60 Monate | praktisch unverändert | | 95,3 mg | 2,4 mg | 1,0 mg |
| (Deg. = Degradation) | | | | | |

**Patentansprüche**

1. Leicht redispergierbare und chemisch stabile pharmazeutische Zubereitung in Form von Cefixim enthaltenden nichtwäßrigen Suspensionen, dadurch gekennzeichnet, daß sie

   a) frei von verdickenden Hilfsstoffen sind,
   b) einen feinstgepulverten Füllstoff mit einer Korngrößenverteilung von mindestens 80 Gew.-% mit einem maximalen Durchmesser von 32 μm und maximal 5 Gew.-% mit einem Durchmesser größer als 50 μm enthalten,
   c) 0,1 bis 5 Gew.-% des Wirkstoffes in mikronisierter Form enthalten, der in Triglyceriden mittelkettiger Fettsäuren suspendiert ist,
   d) zusätzlich ein Netzmittel in einer Konzentration zwischen 0,01 und 1 Gew.-% enthalten.

**Claims**

1. Easily redispersible and chemically stable pharmaceutical composition in the form of cefixime-containing non-aqueous suspensions, characterized in that they

   a) are free of thickening ancillary substances,
   b) contain a microfine powder of bulking agent with a particle size distribution of at least 80% by weight having a maximum diameter of 32 μm and a maximum of 5% by weight having a diameter larger than 50 μm,
   c) contain 0.1 to 5% by weight of the active substance in micronized form which is suspended in triglycerides of medium chain-length fatty acids,
   d) additionally contain a wetting agent in a concentration between 0.01 and 1% by weight.

**Revendications**

1. Préparation pharmaceutique facilement redispersable et chimiquement stable sous forme de suspensions non aqueuses contenant du céfixime, caractérisée

   a) en ce qu'elles ne contiennent pas d'adjuvants épaississants,
   b) en ce qu'elles contiennent une matière de charge très finement pulvérisée présentant une granulométrie telle qu'au moins 80 % en poids de la matière de charge a un diamètre maximal de 32 μm et qu'au plus 5 % en poids a un diamètre supérieur à 50 μm,
   c) en ce qu'elles contiennent 0,1 à 5 % en poids de la substance active sous forme micronisée, mise en suspension dans des triglycérides d'acides gras à chaîne moyenne,
   d) en ce qu'elles contiennent en plus un agent mouillant à une concentration comprise entre 0,01 et 1 % en poids.